(19) Europäisches Patentamt
European Patent Office
Office européen des brevets

(11) Veröffentlichungsnummer: **0 005 484**
**B1**

(12) # EUROPÄISCHE PATENTSCHRIFT

(21) Anmeldenummer: **79101362.6**

(22) Anmeldetag: **04.05.79**

(51) Int. Cl.$^3$: **C 07 C 120/00,**
C 07 C 121/00

(54) **Verfahren zur Herstellung von Acylcyaniden**

(30) Priorität: **11.05.78 DE 2820575**

(43) Veröffentlichungstag der Anmeldung:
**28.11.79 Patentblatt 79/24**

(45) Bekanntmachung des Hinweises auf die Patenterteilung:
**10.12.80 Patentblatt 80/25**

(84) Benannte Vertragsstaaten:
**BE CH DE FR GB IT NL**

(56) Entgegenhaltungen:
**CH - A - 435 235**
**DE - A - 2 614 240**
**DE - A - 2 614 241**
**DE - A - 2 614 242**
**DE - B - 1 266 751**
**GB - A - 1 247 929**
**US - A - 2 426 014**

(73) Patentinhaber: **BAYER AG**
**Zentralbereich Patente, Marken und Lizenzen**
**D - 5090 Leverkusen 1, Bayerwerk (DE)**

(72) Erfinder: **Findeisen, Kurt, Dr.**
**In der Follmühle 10**
**D - 5068 Odenthal 2 (DE)**
**Linker, Karl-Heinz**
**Albert-Schweitzer-Strasse 3**
**D - 5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 005 484 B1

Courier Press, Leamington Spa, England.

# Verfahren zur Herstellung von Acylcyaniden

Die vorliegende Erfindung betrifft ein chemisch eigenartiges Verfahren zur Herstellung von monomeren Acylcyaniden aus dimeren Acylcyaniden; Acylcyanide können als Zwischenprodukte zur Herstellung von Herbiziden verwendet werden.

Es ist bereits seit langem bekannt, daß monomere Acylcyanide unter dem Einfluß von metallischem Natrium (vgl. J. prakt.Chem. (2), Bd. 39, S. 260 (1889)) oder Ätzkali zu entsprechend substituierten O-Acyl-tartronsäuredinitrilen ("dimeren Acylcyaniden") dimerisiert werden (Liebigs Ann.Chem. *120*, S. 334 (1861)).

Ferner ist bekannt, daß auch bei der Umsetzung bestimmter Carbonsäure-Derivate wie Carbonsäurechloride oder-anhydride mit Blausäure in Anwesenheit basischer Katalysatoren dimere Acylcyanide gebildet werden (vgl. Angew. Chem. *68*, S. 434—435 (1956)). Die Bildung von — in diesem Falle unerwünschten — dimeren Acylcyaniden läßt sich häufig auch bei der Synthese der monomeren Acylcyanide nicht unterdrücken und führt dann zu mehr oder weniger hohen Ausbeuteverlusten an monomerem Acylcyanid (vgl. Angew.Chem. *68*, S. 425—426 (1956)).

Es wurde nun überraschend gefunden, daß man die monomeren Acylcyanide der Formel

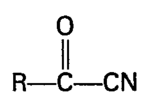 (I),

in welcher

R für gegebenenfalls substituiertes Alkyl mit bis zu 8 Kohlenstoffatomen, gegebenenfalls substituiertes Cycloalkyl mit 3 bis 12 Kohlenstoffatomen, gegebenenfalls substituiertes Aryl oder für einen gegebenenfalls substituierten 5- oder 6-gliedrigen heterocyclischen Rest steht, der zusätzlich noch mit einem Benzolring anelliert sein kann,

in sehr hoher Ausbeute und ausgezeichneter Reinheit erhält, wenn man die entsprechenden dimeren Acylcyanide der Formel

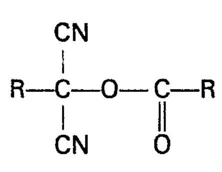 (II),

in welcher

R die oben angegebene Bedeutung hat,

in Gegenwart von basisch reagierenden Verbindungen und gegebenenfalls in Gegenwart eines Verdünnungsmittels auf Temperaturen von 50 bis 300°C erhitzt und die gebildeten monomeren Acylcyanide (I) durch Destillation, gegebenenfalls unter vermindertem Druck, aus dem Reaktionsmedium entfernt. Wie außerdem gefunden wurde, können die dimeren Acylcyanide (II) auch in Mischung mit den jeweils entsprechenden monomeren Acylcyaniden (I) eingesetzt werden; solche Gemische erhält man, wenn man nach vorbekannten Verfahren Carbonsäure-Derivate mit Blausäure in Anwesenheit basischer Katalysatoren umsetzt (vgl. Angew.Chem. *68*, S. 425—4488) (1956).

Es ist im Hinblick auf den Stand der Technik als ausgesprochen überraschend zu bezeichnen, daß momomere Acylcyanide der Formel (I) nach dem erfindungsgemäßen Verfahren durch Spaltung der entsprechenden dimeren Acylcyanide (II) in hoher Ausbeute und ausgezeichneter Reinheit zugänglich sind, denn es war bekannt, daß monomere Acylcyanide in Gegenwart von basischen Katalysatoren umgekeht in dimere Acylcyanide umgewandelt werden; die Umkehrung dieser Reaktion, d.h. die glatte Spaltung der dimeren zu monomeren Acylcyaniden unter sehr ähnlichen Bedingungen, konnte keinesfalls erwartet werden. Insbesondere war auch nicht vorherzusehen, daß die Bildung von harzartigen Produkten beim Erwärmen des Reaktionsgemisches in Gegenwart basisch reagierender Verbindungen unterbleibt (vgl. Liebigs Annalen der Chemie *287*, S. 306 (1895)).

Das erfindungsgemäße Verfahren besitzt eine Reihe von Vorteilen. So ist es nicht auf die Synthese weniger bestimmter Verbindungen beschränkt, sondern es läßt sich sehr breit anwenden. Weiterhin liefert das erfindungsgemäße Verfahren Acylcyanide in praktisch quantitativer Ausbeute und ausgezeichneter Reinheit, frei von störenden oder umweltfeindlichen Nebenprodukten. Ein weiterer wesentlicher Vorteil des neuen Verfahrens besteht darin, daß die Aufarbeitung keine Probleme bietet: die gewünschten monomeren Acylcyanide wurden durch Destillation aus dem Reaktionsgemisch unmittelbar rein erhalten.

Ein zusätzlicher, ganz entscheidender Vorteil des erfindungsgemäßen Verfahrens besteht darin, daß die dimeren Acylcyanide (II) nicht in reiner Form eingesetzt werden müssen, sondern in Mischung mit dem jeweils entsprechenden monomeren Acylcyanid (I) eingesetzt werden können. Solche (I)-(II)-Gemische werden erhalten, wenn man [mit dem Ziel, monomere Acylcyanide herzustellen] nach dem Stand der Technik die entsprechenden Acylchloride mit Blausäure und Pyridin als säurebindendem Mittel zur Reaktion bringt (vgl. J.Chem.Soc. [London] *127*, S. 1635 (1925)). Der Anteil an dimerem Acylcyanid (II), das bei dem vorbekannten Verfahren als unerwünschtes Nebenprodukt auftritt, kann sehr beträchtlich sein (bis zu ca. 70%) und vermindert dementsprechend

die Ausbeute an monomerem Acylcyanid (I). Schließt man nun an das vorbekannte Verfahren vor der destillativen Trennung des dabei stets erhaltenen Monomer-/Dimer-Gemisches das erfindungsgemäße Verfahren an, so gelingt es, auch Acylchloride und andere Säurederivate — ohne Zwischenisolierung der dimeren Acylcyanide (II) — mit ausgezeichneten Ausbeuten in monomere Acylcyanide (I) zu überführen.

Verwendet man das dimere Benzoylcyanid als Ausgangsprodukt und Natriumcyanid als Katalysator, so kann der Reaktionsverlauf des erfindungsgemäßen Verfahrens durch das folgende Formelschema wiedergegeben werden:

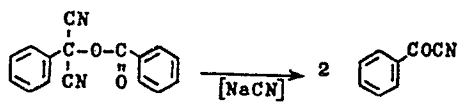


Die als Ausgangsstoffe verwendeten dimeren Acylcyanide sind durch die Formel (II) allgemein definiert. In dieser Formel steht R vorzugsweise für geradkettiges oder verzweigtes Alkyl mit 2 bis 5 Kohlenstoffatomen, wobei jeder dieser Alkylreste substituiert sein kann durch (gegebenenfalls substituiertes) Aryl mit 6 bis 10 Kohlenstoffatomen, vorzugsweise Phenyl (wobei als Substituenten die gleichen in Frage kommen wie unten für R = Aryl angegeben), durch Alkoxy mit 1 bis 4 Kohlenstoffatomen, Carbalkoxy mit 1 bis 4 Kohlenstoffatomen in der Alkoxygruppe, Nitro, Nitril und/oder Halogen, wie z.B. Fluor, Chlor, Brom oder Jod. Ferner steht R vorzugsweise für gegebenenfalls durch Alkyl, Alkoxy oder Carbalkoxy mit jeweils bis zu 4 Kohlenstoffatomen, Nitro, Nitril und/oder Halogen, wie z.B. Fluor, Chlor und Brom, substituiertes Cycloalkyl mit 5 oder 6 Kohlenstoffatomen im Ringsystem. Weiterhin steht R vorzugsweise für gegebenenfalls durch Alkyl, Alkoxy oder Carbalkoxy mit jeweils bis zu 4 Kohlenstoffatomen, Nitro, Nitril und/oder Halogen, wie z.B. Fluor, Chlor und Brom, substituiertes Aryl, insbesondere Phenyl oder Naphthyl. Schließlich steht R vorzugsweise für gegebenenfalls durch Alkyl, Alkoxy oder Carbalkoxy mit jeweils bis zu 4 Kohlenstoffatomen, Nitro, Nitril und/oder Halogen, wie z.B. Fluor, Chlor und Brom, substituierte 5- oder 6-gliedrige heterocyclische Reste, die 1 bis 3 Heteroatomen wie Sauerstoff, Schwefel und/oder Stickstoff im Ring enthalten können und außerdem mit einem Benzolring anelliert sein können. Als Beispiele für insbesondere in Frage kommende heterocyclische Reste seien genannt:

Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Die als Ausgangsstoffe verwendeten dimeren Acylcyanide sind bekannt oder lassen sich nach bekannten Verfahren herstellen (Angew.Chemie *68*, S. 425—435 (1956); siehe auch unter Herstellungsbeispielen).

Als bevorzugte Beispiele für dimere Acylcyanide der Formel (II) seien im einzelnen die Dimeren der folgenden Acylcyanide genannt:

Acetylcyanid, Propionylcyanid, Pivaloylcyanid, Cyclohexancarbonsäurecyanid, Cyclopentancarbonsäurecyanid, Benzoylcyanid, m-Chlorbenzoylcyanid, 3,5-Dichlorbenzoylcyanid, Naphthalin - 1 - carbonsäurecyanid, 1 - Phenyl - 5 - pyrazolon - 3 - carbonsäurecyanid. Als besonders bevorzugte Ausgangsverbindungen seien das dimere Pivalolylcyanid und das dimere Benzoylcyanid genannt.

Als Beispiele für erfindungsgemäß einzusetzende, basisch reagierende Verbindungen seien im einzelnen folgende Stoffe genannt:

Alkalisalze, insbesondere Natrium- und Kaliumsalze von aliphatischen, cycloaliphatischen und aromatischen Carbonsäuren, z.B. Natriumacetat, Natriumbenzoat, sowie die Natriumsalze der Pivalinsäure und der Cyclohexylglyoxylsäure; Alkalicyanide und komplexe Cyanide, wie beispielsweise Natriumcyanid, Kaliumcyanid, Natrium-Zinkcyanid, Kalium-Zinkcyanid, Natrium- und Kalium-Kupfercyanid; tertiäre Amine, wie z.B. Triäthylamin, Dimethylbenzylamin, 1.4 - Diazabicyclooctan - (2.2.2), 1.8 - Diazabicyclo - (5.4.0) - undec - 7 - en, 1.5 - Diaza - bicyclo - (4.3.0) - non - 5 - en, Pyridin und Chinolin; Alkali- und Erdalkalihydroxide, wie Natriumhydroxid, Kaliumhydroxid, Calciumhydroxid und Bariumhydroxid; ferner Alkoholate und Phenolate, wie beispielsweise Natriummethylat, Natriumäthylat, Kalium - tert. - butylat und Natriumphenolat.

Besonders bevorzugte Basen sind Natrium- und Kaliumcyanid, Natrium- und Kaliumacetat, Natrium- und Kaliumbenzoat sowie das Natrium- und Kaliumsalz der Pivalinsäure.

Als Verdünnungsmittel, die bei der Durchführung des erfindungsgemäßen Verfahrens eingesetzt werden können, kommen alle inerten organischen Lösungsmittel, die mit den Acylcyaniden keine chemische Reaktion eingehen und mindestens 20°C höher als das entstehende Acylcyanid sieden, in Betracht. Solche Lösungsmittel sind beispielsweise die Xylole wie o-Xylol, Chlorbenzol, o-Dichlorbenzol, die Trichlorobenzole, Nitrobenzol, Tetramethylensulfon, Hexamethylphosphorsäuretriamid, Benzonitril, Benzylcyanid, Acetanhydrid, Pivalinsäureanhydrid, Cyclohexancarbonsäureanhydrid, Benzosäureanhydrid, Benzosäuremethylester.

Prinzipiell ist es jedoch auch möglich, die erfindungsgemäße Umsetzung ohne Verdünnungsmittel durchzuführen.

Die Reaktionstemperatur kann in einem größeren Bereich variiert werden. Im allgemeinen arbeitet man bei Temperaturen zwischen 50 und 300°C, vorzugsweise zwischen 80 und 250°C, insbesondere zwischen 100 und 220°C. Am zweckmäßigsten ist es, unterhalb des Siedepunktes des zu spaltenden dimeren Acylcyanids und oberhalb des Siedepunktes des monomeren Acylcyanids zu arbeiten.

Das erfindungsgemäße Verfahren wird je nach dem Siedepunkt des gebildeten monomeren Acylcyanids entweder bei Normaldruck oder bei vermindertem Druck durchgeführt. Es ist zu empfehlen, daß höher siedende Acylcyanide durch Vakuumdestillation aus dem Reaktionsgemisch entfernt werden. In diesen Fällen arbeitet man im Druckbereich bis etwa 0,1 mbar.

Bei der Durchführung des erfindungsgemäßen Verfahrens setzt man die basisch reagierenden Verbindungen in katalytischen Mengen ein, auf 1 Mol dimeres Acylcyanid (II) im allgemeinen 0,01 bis 0,2 Mol, vorzugsweise 0,05 bis 0,1 Mol einer basisch reagierenden Verbindung.

Das Verfahren wird am zweckmäßigsten so durchgeführt, daß man das Reaktionsgemisch bei Normaldruck oder entsprechend vermindertem Druck — je nach dem Siedepunkt des herzustellenden monomeren Acylcyanids der Formel (I) — auf die Reaktionstemperatur erhitzt. Hierbei wird das gebildete Acylcyanid laufend aus dem Reaktionsgemisch abdestilliert und dadurch isoliert. Eine weitere Reinigung ist in den meisten Fällen nicht erforderlich; jedoch können die erhaltenen Acylcyanide gewünschtenfalls re-destilliert und/oder umkristallisiert werden.

Wenn man nicht von reinem dimerem Acylcyanid (II) ausgeht, sondern in literaturbekannter Weise zunächst ein Gemisch aus monomerem und dem entsprechenden dimeren Acylcyanid herstellt, so ist es am zweckmäßigsten, vor der Destillation eine katalytische Menge einer basisch reagierenden Verbindung zuzufügen und dann (gegebenenfalls nach Entfernen des Verdünnungsmittels) auf die Reaktions- bzw. Siedetemperatur zu erhitzen, gegebenenfalls unter vermindertem Druck. Reines monomeres Acylcyanid (I) wird wiederum durch Destillation isoliert.

In einer besonderen Verfahrensvariante läßt sich die erfindungsgemäße Umsetzung auch kontinuierlich gestalten.

Die nach dem erfindungsgemäßen Verfahren herstellbaren Acylcyanide der Formel (I) sind wertvolle Ausgangsstoffe z.B. zur Synthese von 1,2,4-Triazin-5-onen, welche hervorragende herbizide Eigenschaften besitzen (vgl. Deutsche Offenlegungsschrift 2 224 161).

So läßt sich beispielsweise das 3 - Methyl - 4 - amino - 6 - phenyl - 1,2,4 - triazin - 5 - on der Formel

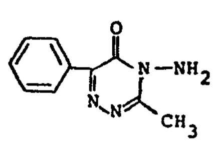

herstellen, indem man in einer ersten Stufe Benzoylcyanid in Gegenwart von konzentrierter Salzsäure mit Äthanol umsetzt und den dabei entstehenden Phenylglyoxylsäureäthylester in einer zweiten Stufe mit Acetylhydrazin zur Reaktion bringt, wobei sich 1 - Phenylglyoxylsäureäthylester - 2 - acetylhydrazon bildet, das in einer dritten Stufe mit Hydrazinhydrat in Gegenwart von Pyridin in das oben erwähnte Endprodukt überführt wird. Diese mehrstufige Synthese läßt sich formelmäßig wie folgt wiedergeben:

1. Stufe:

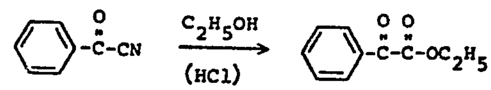


2. Stufe

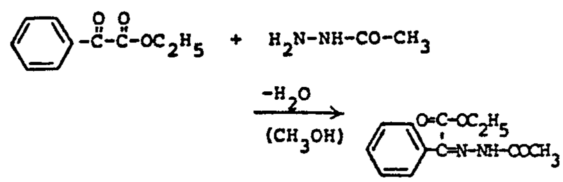


3. Stufe

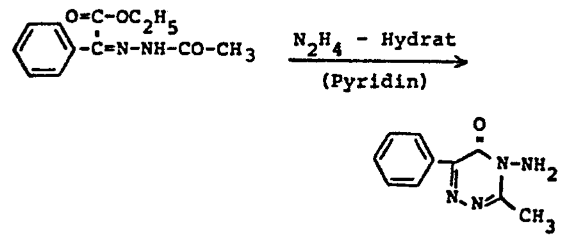


Das erfindungsgemäße Verfahren wird durch die nachfolgenden Herstellungsbeispiele veranschaulicht:

*Herstellungsbeispiele*

Beispiel 1

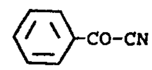

In einem mit Rührer, Thermometer und einer kleinen Destillationskolonne mit Destillationsaufsatz versehenen 250 ml-Dreihalskolben wurden 131 g dimeres Benzoylcyanid (0,5 Mol) mit 2 g Natriumcyanid versetzt und im Wasserstrahlvakuum (16 mbar) langsam erwärmt. Bei 160 bis 165°C Innentemperatur begann das monomere Benzoylcyanid in die Vorlage zu destillieren. Die Innentemperatur wurde langsam auf 200°C gesteigert, wobei weiteres Benzoylcyanid abdestillierte (Kopftemperatur 92 bis 130°C/16 mbar).

Ausbeute: 122 g reines Benzoylcyanid (93% der Theorie);
Siedpunkt: 87 bis 89°C bei 17,3 mbar;
Schmelzpunkt: 32°C.

Beispiel 2

Analog zu Beispiel 1 wurden 131 g dimeres

Benzoylcyanid (0,5 Mol) mit 3 g Kaliumcyanid versetzt. Die Umsetzung und Aufarbeitung erfolgte ebenfalls entsprechend Beispiel 1.
Ausbeute: 123 g reines Benzoylcyanid (94% der Theorie);
Schmelzpunkt: 32°C.

Beispiel 3

Analog zu Beispiel 1 wurden in einem entsprechenden 500 ml-Kolben 131 g dimeres Benzoylcyanid (0,5 Mol) und 5 g Natriumbenzoat in 113 g Benzoesäureanhydrid (0,5 Mol) gelöst und unter Wasserstrahlvakuum (16 mbar) auf 160°C erwärmt. Hierbei begann das Benzoylcyanid abzudestillieren. Die Innentemperatur wurde langsam auf 210°C gesteigert, wobei weiteres Benzoylcyanid abdestillierte.
Ausbeute: 127 g reines Benzoylcyanid (97% der Theorie);
Schmelzpunkt: 32°C.

Beispiel 4

Analog zu Beispiel 1 wurden in einem entsprechenden 500 ml-Kolben 262 g dimeres Benzoylcyanid (1 Mol) mit 3 g 1,4 - Diaza - bicyclooctan - (2,2,2) versetzt. Die Umsetzung und Aufarbeitung erfolgte ebenfalls entsprechend Beispiel 1.

Ausbeute: 234 g reines Benzoylcyanid (89% der Theorie);
Schmelzpunkt: 32°C.

*Vergleichsbeispiel zu Beispielen 1 bis 4*

262 g dimeres Benzoylcyanid (1 Mol) wurden ohne Zusatz einer basischen Verbindung 2 Stunden auf 200°C erwärmt. Die Reaktionsmischung wurde anschließend im Vakuum destilliert.
Ausbeute: 258 g dimeres Benzoylcyanid (= 98% der Theorie);
Siedepunkt: 163 bis 164°C bei 0,4 mbar;
Schmelzpunkt: 96 bis 97°C (aus Methanol).

Beispiel 5

Entsprechend den Angaben in der Literatur (J.Chem.Soc. [London] *127,* S. 1635 (1925)) wurden 140,6 g Benzoylchlorid (1 Mol), 160 ml Blausäure (4 Mol) in 1500 ml trockenem Äther in einem 3 Liter-Kolben vorgelegt. Bei 0 bis 10°C wurden 316,4 g wasserfreies Pyridin (4 Mol) unter Rühren innerhalb von 1 Stunde zugetropft. Anschließend wurde das Reaktionsgemisch noch 12 Stunden bei 0 bis 10°C gehalten. Das ausgefallene Pyridinhydrochlorid wurde abgesaugt und mit Äther gewaschen. Die Ätherlösungen wurden eingeengt und von geringen Mengen eines Niederschlages abfiltriert. Nach Abziehen des Lösungsmittels wurde das zurückbleibende Reaktionsprodukt mit 5 g Natriumcyanid versetzt und dann im Wasserstrahlvakuum langsam auf 160 bis 165°C, später auf 200 bis 210°C, erhitzt, wobei schließlich die Gesamtmenge Benzoylcyanid abdestillierte.

Ausbeute: 118 g reines Benzoylcyanid (90% der Theorie, bezogen auf Benzoylchlorid);
Siedepunkt: 92 bis 95°C bei 18,6 mbar;
Schmelzpunkt: 32°C.

*Vergleichsbeispiel zu Beispiel 5*

In einem Vergleichsversuch wurde ein zweiter Ansatz unter den gleichen Bedingungen wie in Beispiel 5 beschrieben durchgeführt. Das durch die Umsetzung von Benzoylchlorid mit Blausäure und Pyridin in Äther erhaltene, nach Abziehen des Lösungsmittels zurückbleibende Reaktionsprodukt wurde jedoch nicht mit Natriumcyanid versetzt, sondern wurde einer fraktionierten Destillation unterworfen, um das im Reaktionsprodukt enthaltene Gemisch von monomerem und dimerem Benzoylcyanid abzutrennen.

Ausbeuten:

I) 80 g monomeres Benzoylcyanid (61% der Theorie),
Siedepunkt: 92 bis 95°C bei 18,6 mbar;

II) 39,1 g dimeres Benzoylcyanid (32% der Theorie),
Siedepunkt: 163 bis 164°C bei 0,4 mbar.

Beispiel 6

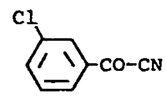

Analog zu Beispiel 1 wurden 166 g dimeres 3-Chlorbenzoylcyanid (0,5 Mol) in Gegenwart von 3 Natriumcyanid auf 180°C erhitzt. Durch Verminderung des Druckes auf 16 mbar begann das monomere 3-Chlorbenzoylcyanid aus dem Reaktionsgemisch überzudestillieren. Während der Destillation wurde die Außentemperatur langsam auf 210°C gesteigert, um die Spaltung des Dimeren zu vervollständigen.

Ausbeute: 154 g reines 3-Chlorbenzoylcyanid (93% der Theorie);
Siedepunkt: 118 bis 120°C bei 18,6 mbar;
Schmelzpunkt: 146 bis 148°C
(aus Waschbenzin).

Beispiel 7

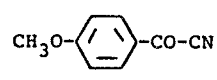

Analog zu Beispiel 1 wurden 292 g dimeres 4-Methoxybenzoylcyanid (0,5 Mol) in Gegenwart von 3 g Natriumcyanid unter Wasserstrahlvakuum (16 mbar) zunächst langsamt auf 180°C, gegen Ende der Umsetzung auf 210°C erhitzt. Hierbei destillierte das gebildete monomere 4-Methoxybenzoylcyanid langsam über und erstarrte in der Vorlage.

Ausbeute: 258 g reines 4-Methoxybenzoylcyanid (88% der Theorie);
Schmelzpunkt: 61 bis 63°C.

Beispiel 8

$(CH_3)_3C—CO—CN$

Analog zu Beispiel 1 wurden in einem entsprechenden 500 ml-Kolben 222 g dimeres Pivaloylcyanid (1 Mol) mit 5 g Natriumacetat versetzt und bei Normaldruck zunächst auf 150°C, anschließend auf 200°C erhitzt. Hierbei destillierte das entstehende monomere Pivaloylcyanid ab.

Ausbeute: 214 g reines Pivaloylcyanid (96% der Theorie);
Siedepunkt: 121 °C (bei Normaldruck).

Beispiel 9

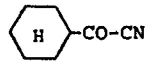

Analog zu Beispiel 1 wurden 137 g dimeres Cyclohexanoylcyanid (= Cyclohexylglyoxylsäure-nitril) (0,5 Mol) und 5 g Kaliumacetat in 200 g Cyclohexancarbonsäureanhydrid unter Wasserstrahlvakuum (18,6 mbar) langsam auf 140 bis 170°C erhitzt, wobei das gebildete monomere Cyclohexanoylcyanid abdestillierte. Nach einer weiteren Reinigung durch anschließende fraktionierte Destillation betrug die. Ausbeute: 119 g reines Cyclohexanoylcyanid (87% der Theorie);
Siedepunkt: 79 bis 82°C bei 18,6 mbar.

*Beispiele für die Herstellung von Ausgangsprodukten*

A) Dimeres Benzoylcyanid

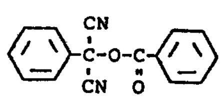

gemäß den Angaben in der Literatur (Ber.dtsch.chem. ges. *41*, S. 1896 (1908)) wurden 160 g wasserfreie Blausäure (5,92 Mol) bei 10 bis 15°C unter Rühren in 500 ml wasserfreies Pyridin (6,32 Mol) eingetropft, dann wurden unter Kühlung 832 g Benzoylchlorid (5,92 Mol) tropfenweise zugegeben. Die Reaktionsmischung wurde 1 Stunde nachgerührt und dann über Nacht stehen gelassen. Zu der festen Mischung wurden 2 l 30%ige Schwefelsäure zugegeben; der verbleibende, darin unlösliche Rückstand wurde abgesaugt und mit 800 ml 30%iger Schwefelsäure gewaschen. Der Filterrückstand wurde aus 2,5 l Methanol umkristallisiert.

Ausbeute: 552 g dimeres Benzoylcyanid (71% der Theorie);
Schmelzpunkt: 96 bis 97°C.
B) Dimeres Pivaloylcyanid

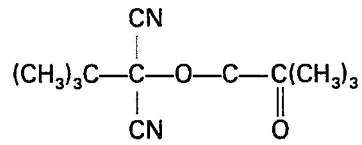

186 g Pivalinsäureanhydrid (1 Mol) und 49 g Natriumcyanid wurden gemischt und langsam auf 120°C erwärmt, wobei eine schwach exotherme Reaktion einsetzte. Das Reaktionsgemisch wurde zähflüssig.

Anschließend wurde das Reaktionsgemisch 1 Stunde auf 150°C geheizt, dann abgekühlt und mit 300 ml Xylol versetzt. Eine geringe Menge Unlösliches wurde abfiltriert, und das Filtrat wurde fraktioniert destilliert.

Ausbeute: 91 g dimeres Pivaloylcyanid (82% der Theorie);
Siedepunkt: 136 bis 137°C bei 20 mbar;
Schmelzpunkt: 54,5 bis 55,4°C (aus Isopropanol).

**Patentansprüche**

1. Verfahren zur Herstellung von monomeren Acylcyaniden der Formel

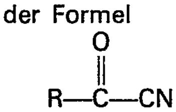

(I),

in welcher
R für gegebenenfalls substituiertes Alkyl mit bis zu 8 Kohlenstoffatomen, gegebenenfalls substituiertes Cycloalkyl mit 3 bis 12 Kohlenstoffatomen, gegebenenfalls substituiertes Aryl oder für einen gegebenenfalls substituierten 5- oder 6- gliedrigen heterocyclischen Rest steht, der zusätzlich noch mit einem Benzolring anelliert sein kann,
dadurch gekennzeichnet, daß man die entsprechenden dimeren Acylcyanide (substituierte O-Acyl-tartronsäuredinitrile) der Formel

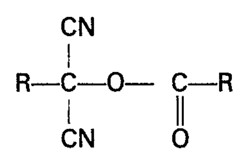

(II),

in welcher
R die oben angegebene Bedeutung hat,

in Gegenwart von basisch reagierenden Verbindungen und gegebenenfalls in Gegenwart eines Verdünnungsmittels auf Temperaturen von 50 bis 300°C erhitzt und die gebildeten monomeren Acylcyanide (I) durch Destillation, gegebenenfalls unter vermindertem Druck, aus dem Reaktionsmedium entfernt.

2. Verfahren gemäß Anspruch 1, dadurch gekennzeichnet, daß die Emsetzung bei Temperaturen zwischen 80 und 250°C durchgeführt wird.

3. Verfahren gemäß Anspruch 1, dadurch gekennzeichnet, daß die Umsetzung bei Temperaturen zwischen 100 und 220°C durchgefürt wird.

4. Verfahren gemäß Anspruch 1, dadurch gekennzeichnet, daß die Umsetzung bei vermindertem Druck, vorzugsweise im Druckbereich bis 0,1 mbar durchgeführt wird.

5. Verfahren gemäß Anspruch 1, dadurch gekennzeichnet, daß die basisch reagierenden Verbindungen in katalytischen Mengen eingesetzt werden.

6. Verfahren gemäß Anspruch 5, dadurch gekennzeichnet, daß auf 1 Mol dimeres Acylcyanid (II) 0,01 bis 0,2 Mol, vorzugsweise 0,05 bis 0,1 Mol einer basisch reagierenden Verbindung eingesetzt werden.

7. Verfahren gemäß Anspruch 1, dadurch gekennzeichnet, daß man die dimeren Acylcyanide der Formel (II) Form von Gemischen mit den jeweils entsprechenden monomeren Acyl-cyaniden der Formel (I), erhalten durch Umsetzung von Carbonsäurederivaten mit Blausäure in Gegenwart basischer Katalysatoren nach vorbekannten Verfahren, einsetzt.

8. Verfahren gemäß Anspruch 1, dadurch gekennzeichnet, daß man als dimere Acylcyanide der Formel (II) dimeres Benzoylcyanid, dimeres Pivaloylcyanid oder dimeres Cyclohexanoylcyanid einsetzt.

9. Verfahren gemäß Anspruch 1, dadurch gekennzeichnet, daß man als basisch reagierende Verbindungen Natriumcyanid, Kaliumcyanid, Natriumacetat, Kaliumacetat oder Natriumbenzoat einsetzt.

10. Verfahren gemäß Anspruch 1, dadurch gekennzeichnet, daß man als Verdünnungsmittel Acetanhydrid, Pivalinsäureanhydrid, Benzoesäureanhydrid oder Cyclohexancarbonsäureanhydrid einsetzt.

## Claims

1. Process for the preparation of monomeric acyl cyanides of the formula

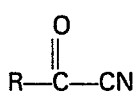 (I)

in which

R represents optionally substituted alkyl with up to 8 carbon atoms, optionally substituted cycloalkyl with 3 to 12 carbon atoms, optionally substituted aryl or an optionally substituted 5-membered or 6-membered heterocylic radical, which additionally can also be fused with a benzene ring.

characterised in that the corresponding dimeric acyl cyanides (substituted O-acyl-tartronic acid dinitriles) of the formula

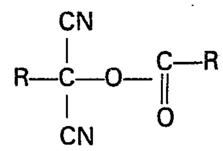 (II)

in which

R has the meaning indicated above,

are heated to temperatures from 50 to 300°C in the presence of compounds having a basic reaction and if appropriate in the presence of a diluent, and the monomeric acyl cyanides (I) formed are removed from the reaction medium by distillation, if appropriate under reduced pressure.

2. Process according to Claim 1, characterised in that the reaction is carried out at temperatures between 80 and 250°C.

3. Process according to Claim 1, characterised in that the reaction is carried out at temperatures between 100 and 220°C.

4. Process according to Claim 1, characterised in that the reaction is carried out under reduced pressure, preferably in the pressure range down to 0.1 mbars.

5. Process according to Claim 1, characterised in that the compounds having a basic reaction are employed in catalytic amounts.

6. Process according to Claim 5, characterised in that 0.01 to 0.2 mol, preferably 0.05 to 0.1 mol, of a compound having a basic reaction is employed per 1 mol of dimeric acyl cyanide (II).

7. Process according to Claim 1, characterised in that the dimeric acyl cyanides of the formula (II) are employed in the form of mixtures with the particular corresponding monomeric acyl cyanides of the formula (I), obtained by reacting carboxylic acid derivatives with hydrocyanic acid in the presence of basic catalysts by previously known processes.

8. Process according to Claim 1, characterised in that dimeric benzoyl cyanide, dimeric pivaloyl cyanide or dimeric cyclohexanoyl cyanide are employed as the dimeric acyl cyanides of the formula (II).

9. Process according to Claim 1, characterised in that sodium cyanide, potassium cyanide, sodium acetate, potassium acetate or sodium benzoate are employed as the compounds having a basic reaction.

10. Process according to Claim 1, characterised in that acetic anhydride, pivalic acid anhydride, benzoic acid anhydride or cyclohexanecarboxylic acid anhydride is employed as the diluent.

## Revendications

1. Procédé de préparation de cyanures d'acyle monomères répondant à la formule

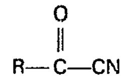 (I)

dans laquelle

R représente un groupe alkyle éventuellement substitué contenant jusqu'à 8 atomes de carbone, un groupe cycloalkyle éventuellement substitué contenant de 3 à 12 atomes de carbone, un groupe aryle éventuellement substitué ou un reste hétérocyclique à 5 ou 6 chaînons éventuellement substitué et qui peut en outre être condensé sur un cycle benzénique,

ce procédé se caractérisant en ce que l'on chauffe les cyanures d'acyle dimères correspondants (dinitriles d'acides O-acyl-tartroniques substitués) de formule

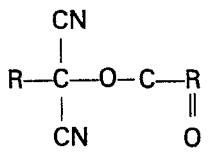

(II)

dans laquelle R a la signification indiquée ci-dessus, en présence de composés à réaction basique et le cas échéant en présence d'un diluant, à des température de 50 à 300°C, et on sépare les cyanures d'acyle monomères formés, répondant à la formule I, du milieu de réaction par distillation, éventuellement sous vide.

2. Procédé selon la revendication 1, caractérisé en ce que la réaction est effectuée à des températures de 80 à 250°C.

3. Procédé selon la revendication 1, caractérisé en ce que la réaction est effectuée à des températures de 100 à 220°C.

4. Procédé selon la revendication 1, caractérisé en ce que la réaction est effectuée sous vide, de préférence sous un vide pouvant atteindre 0,1 mbar.

5. Procédé selon la revendication 1, caractérisé en ce que l'on utilise les composés à réaction basique en quantités catalytiques.

6. Procédé selon la revendication 5, caractérisé en ce que, pour 1 mole de cyanure d'acyle dimère de formule II, on utilise de 0,01 à 0,2 mole, de préférence de 0,5 à 0,1 mole, d'un composé à réaction basique.

7. Procédé selon la revendication 1, caractérisé en ce que l'on met en oeuvre les cyanures d'acyle dimères de formule II sous forme de mélanges avec les cyanures d'acyle monomères correspondants de formule I, ces mélanges ayant été obtenus par réaction de dérivés d'acides carboxyliques avec l'acide cyanhydrique en présence de catalyseurs basiques selon des procédés connus antérieurement.

8. Procédé selon la revendication 1, caractériséa en ce que l'on met en oeuvre en tant que cyanures d'acyle dimères de formule II le cyanure de benzoyle dimère, le cyanure de pivaloyle dimère ou le cyanure de cyclohexanoyle dimère.

9. Procédé selon la revendication 1, caractérisé en ce que les composés à réaction basique mis en oeuvre sont le cyanure de sodium, le cyanure de potassium, l'acétate de sodium, l'acétate de potassium ou le benzoate de sodium.

10. Procédé selon la revendication 1, caractérisé en ce que l'on utilise comme diluants l'anhydride acétique, l'anhydride pivalique, l'anhydride benzoïque au l'anhydride cyclohexane-carboxylique.